# EUROPEAN PATENT APPLICATION

(11) **EP 1 190 732 A1**
(43) Date of publication of application: **27.03.2002**
(21) Application number: 01117743.3
(22) Date of filing: 30.07.2001
(51) Int. Cl.: A61M 27/00, A61M 25/00, A61M 1/00

(54) **Drainage element with collection body having a circular cross-section**

(30) Priority: 20.09.2000 IT MI002050
(71) Applicant: Gibertoni, Lucio, 41037 Mirandola (Modena) (IT)
(72) Inventor: Gibertoni, Lucio, 41037 Mirandola (Modena) (IT)
(74) Representative: Modiano, Guido, Dr.-Ing.

(57) **Abstract**

A drainage element with a collection body (10) having a circular cross-section, which comprises an elongated body (11) provided with a plurality of internal channels (12) provided with surface slits (15) which produce drainage by capillary action. The elongated body enters, at one end, a discharge tube and the surface slits (15) form a spiral pattern with respect to the elongated body.

## Description

The present invention relates to a drainage element with collection body having a circular cross-section.

It is known that in surgical practice the drainage element is the device that is used to evacuate liquids, generally pathological ones, collected in natural or newly formed cavities.

For the correct operation of the drainage, and therefore to allow the drainage element to perform its purpose, such element must be pervious, inclined and proportionate to the quantity and nature of the material to be evacuated.

This basic rule, valid for all kinds of drainage, must be followed strictly, otherwise the procedure is ineffective and severe complications may arise.

Accordingly, drainage is a fundamental step in the treatment of a hematoma, and failure to perform it, or its incorrect execution, is a severe and immediate danger for the patient's life.

The ideal drainage is one which maintains over time, and with continuity, a constant suction and remains in place for the necessary time, at the operator's discretion, without causing interruptions in flow or alterations to the tissues that surround it; accordingly, the drainage element must be biocompatible.

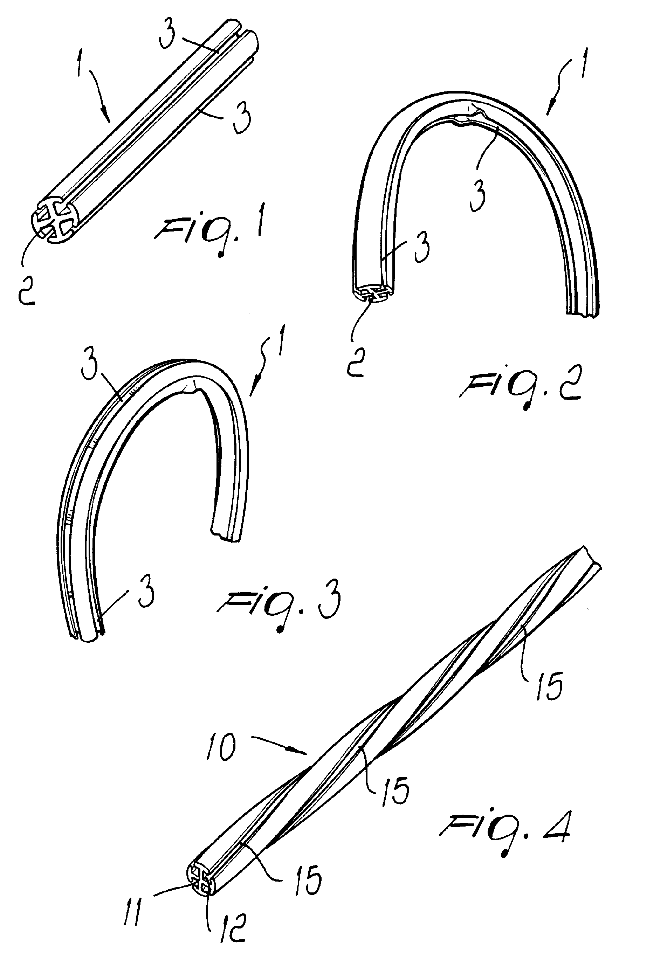

Drainage elements 1, as illustrated in Figure 1, having a circular cross-section are already currently commercially available being internally provided with a plurality of channels which in practice are defined by a central cross-shaped element 2 which forms peripherally, on the circumference of the collection body having a circular cross-section, a plurality of surface slits 3 which lie longitudinally and have a width which allows drainage by capillary action.

Figure 1 illustrates a known type of tube in which there are four longitudinal slits 3 which allow to provide a flow by capillary action which is continuous and constant if the width of the slits remains constant and such as to ensure capillary action.

In practical use, it has been found that quite often the collection body of the drainage element 1 must be bent for optimum placement and this situation causes considerable problems as to the correct operation of the drainage element.

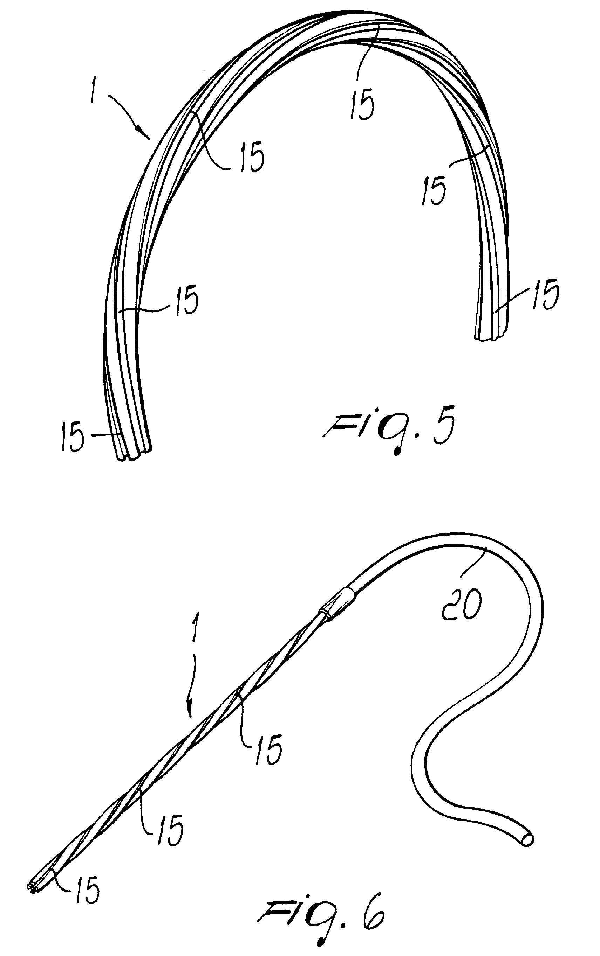

With the solution of the prior art, in the extrados (Figure 3) of the region where bends are formed, the slit, because of the increase in the radius of curvature, tends to open, and therefore capillary action ceases; moreover, in the intrados region (Figure 2) the reduction in the radius of curvature causes compression and therefore splaying of the edges of the slits which again cause a lack of capillary action.

Moreover, the slits located on the lateral walls tend to close, blocking the flow.

Accordingly, in many cases it has been found that drainage ceases to function and in practice the only efficient part of the drainage element is the one that is located downstream of the region occupied by the bend that caused the above described modification of the longitudinal slits that interrupt the capillary action characteristics.

Accordingly, the element loses most of its function, with the obvious consequent risks.

The aim of the invention is to eliminate the above mentioned drawbacks, by providing a drainage element of the type having a collection body with a circular cross-section in which it is possible to prevent the loss of capillary action of the surface slits also in the regions where bends form in the drainage element.

Within this aim, an object of the invention is to provide a drainage element which can be placed in situ according to conventional techniques however always ensuring full and optimum operation of the entire collection body of the drainage element.

Another object of the present invention is to provide a drainage element which, thanks to its particular constructive characteristics, is capable of giving the greatest assurances of reliability and safety in use.

Another object of the present invention is to provide a drainage element which can be easily obtained starting from commonly commercially available elements and materials and is also competitive from a merely economical point of view.

This aim and these and other objects which will become better apparent hereinafter are achieved by a drainage element with a collection body having a circular cross-section, according to the invention, which comprises an elongated body provided with a plurality of internal channels provided with surface slits which produce drainage by capillary action, said elongated body entering, at one end, a discharge tube, characterized in that said surface slits form a spiral pattern with respect to said elongated body.

Further characteristics and advantages of the present invention will become better apparent from the description of a preferred but not exclusive embodiment of a drainage element with a collection body having a circular cross-section, illustrated only by way of non-limitative example in the accompanying drawings, wherein:
Figure 1 is a schematic perspective view of a conventional drainage element;
Figure 2 is a view of the conventional drainage element in a bent position, seen from the intrados;
Figure 3 is a view of the conventional drainage element in a bent position, seen from the extrados;
Figure 4 is a schematic perspective view of a drainage element according to the invention;
Figure 5 is a perspective view of the drainage element according to the invention in a bent position;
Figure 6 is a schematic perspective view of the drainage element connected to the discharge tube.

With reference to the figures and particularly to Figures 4 to 6, the drainage element according to the invention comprises a collection body, generally designated by the reference numeral 10, preferably made of silicone and having a circular cross-section with a substantially cross-shaped central body 11 which forms four internal channels 12, each of which is provided with a surface slit 15 arranged in a spiral pattern with respect to the collection body.

As a result of the spiral arrangement of the surface slits 15, the cross-section remains substantially unchanged even in the region where the collection body 10 is bent, thus tending to maintain its capillary effect.

At one of its ends, the collection body 10 is inserted in a discharge tube, designated by the reference numeral 20.

It has been found experimentally that thanks to the spiral arrangement of the longitudinal slits, the bending to which the collection body is subjected produces in practice a distribution of the effects on the longitudinal slit, so that the compression regions and the traction regions that continuously alternate in the slit compensate for each other, allowing the edges of the longitudinal slits to maintain a substantially unchanged distance and accordingly maintain their capillary characteristic.

Experimental tests that have been conducted have shown that by providing four turns per linear meter the first positive effects on preservation of capillary action are obtained, and these effects persist until twenty-six turns per linear meter are provided; the best results have been obtained by providing thirteen to seventeen turns per linear meter.

Depending on the different diameters, it has been found that with diameters of 2.3 mm for the collection body, the angle formed between the longitudinal dimension of the slits and the longitudinal axis of the tube must be between 2° and 10°, and that this inclination increases as the diameter increases; it has been found experimentally that with tubes having a diameter of 3.3 mm the inclination must be between 2°30' and 15°; with an outside diameter of the collection body of 5 mm, the inclination was between 3°35' and 22°; and with a collection body having a diameter of 6.33 mm the angle was between 4°35' and 27°.

Experimental tests that have been conducted have shown that as the diameter increases, it is necessary to increase correspondingly the inclination of the slit with respect to the longitudinal dimension, while the number of turns per linear meter that allows to maintain capillary action even in the bending regions remains practically unchanged.

From the above description it is thus evident that the invention achieves the intended aim and objects; in particular, the fact is stressed that the provision of a spiral shape for the surface slits of the various internal channels allows to preserve capillary action and accordingly allows to have a drainage element which is always efficient and effective regardless of the curvature imparted thereto for its optimum placement within the region to be drained.

The invention thus conceived is susceptible of numerous modifications and variations, all of which are within the scope of the appended claims.

All the details may further be replaced with other technically equivalent elements.

In practice, the materials used, so long as they are compatible with the specific use, as well as the contingent shapes and dimensions, may be any according to requirements.

The disclosures in Italian Patent Application No. MI2000A002050 from which this application claims priority are incorporated herein by reference.

Where technical features mentioned in any claim are followed by reference signs, those reference signs have been included for the sole purpose of increasing the intelligibility of the claims and accordingly, such reference signs do not have any limiting effect on the interpretation of each element identified by way of example by such reference signs.

## Claims

1. A drainage element with a collection body (10) having a circular cross-section, comprising an elongated body (11) provided with a plurality of internal channels (12) provided with surface slits (15) which produce drainage by capillary action, said elongated body (11) entering, at one end, a discharge tube (20), **characterized in that** said surface slits (15) form a spiral pattern with respect to said elongated body.

2. The drainage element according to claim 1, **characterized in that** said surface slits (15) form four to twenty-six turns per linear meter of said elongated body (11).

3. The drainage element according to claim 2, **characterized in that** said surface slits (15) form thirteen to seventeen turns per linear meter of said elongated body (11).

4. The drainage element according to claim 1, **characterized in that** the angle formed between the longitudinal dimension of said slits (15) and the longitudinal axis of said elongated body increases as a function of the diameter of said elongated body (11).

5. The drainage element according to one or more of the preceding claims, **characterized in that** said collection body (10) is made of silicone.
